# EUROPÄISCHE PATENTSCHRIFT

(11) **EP 2 932 932 B1**
(45) Veröffentlichungstag und Bekanntmachung des Hinweises auf die Patenterteilung: **06.03.2019**
(21) Anmeldenummer: 14164616.6
(22) Anmeldetag: 14.04.2014
(51) Int. Cl.: A61B 90/30, F21V 5/00, F21V 3/00, F21W 131/202, F21Y 115/10

(54) **Medizinische Leuchte**
Medical lamp
Lampe médicale

(43) Veröffentlichungstag der Anmeldung: 21.10.2015
(73) Patentinhaber: Kaltenbach & Voigt GmbH, 88400 Biberach (DE)
(72) Erfinder: Hackel, André, 88400 Biberach (DE)
(74) Vertreter: Thun, Clemens

(56) Entgegenhaltungen:
- EP-A1- 2 587 128
- EP-A2- 2 469 159
- US-A1- 2011 001 431
- US-A1- 2011 175 551
- US-A1- 2012 243 666

## Beschreibung

Die vorliegende Erfindung betrifft eine medizinische Leuchte gemäß dem Oberbegriff des Anspruchs 1. Insbesondere betrifft die Erfindung eine zahnärztliche Behandlungsleuchte für das intraorale Ausleuchten eines Operationsfelds, welche mindestens eine Beleuchtungseinheit mit Leuchtmitteln und optischen Mitteln zur Erzeugung eines Lichtfelds in einer Objektebene aufweist.

Eine gattungsgemäße Leuchte ist bspw. aus der DE 10 2011 075 753 A1 der Anmelderin bekannt. Bei der hier beschriebenen Leuchte kommen als Leuchtmittel LEDs oder LED-Arrays zum Einsatz, denen verschiedene optische Mittel zugeordnet sind, um letztendlich eine für die Behandlungszwecke geeignete Ausleuchtung des Operationsfelds sicherzustellen. Diese optischen Mittel umfassen dabei bspw. zunächst einen Kollimator zur Strahlbündelung sowie einen im Lichtweg nachgeordneten sog. Kondensor, mit dessen Hilfe die Lichtstrahlen derart umgelenkt und/oder vermischt werden, dass in einer Objektebene das angestrebte Lichtfeld erzielt wird. In der Regel ist zusätzlich zu diesen optischen Mitteln noch eine Abdeckscheibe im Strahlengang vorgesehen, deren Funktion allerdings in erster Linie darin besteht, die optischen und elektronischen Komponenten der Leuchte, also insb. die Leuchtmittel sowie die oben beschriebenen optischen Einheiten vor Verschmutzung und Beschädigungen zu schützen.

Aus dem oben beschriebenen Stand der Technik ist weiterhin bereits bekannt, innerhalb einer medizinischen Leuchte mehrere derartige Beleuchtungseinheiten einzusetzen, die in unterschiedlichen Winkeln angeordnet und derart ausgerichtet sind, dass sich das Gesamtbeleuchtungsfeld aus der Überlagerung mehrerer einzelner Leuchtfelder ergibt, was zur Folge hat, dass die Schattenbildung bspw. durch den behandelnden Zahnarzt minimiert wird. Idealerweise sind dabei die Beleuchtungseinheiten derart ausgerichtet bzw. derart ausgebildet, dass die einzelnen Lichtfelder im Wesentlichen identisch sind, also vollständig überlappen.

Das letztendlich erzeugte Lichtfeld sollte dabei möglichst homogen ausgeleuchtet werden, um auch bei evtl. leichten Bewegungen des Patientenkopfs noch ein gut beleuchtetes Behandlungsfeld zu erhalten. Auf diesem Wege kann vermieden werden, dass bei geringfügigen Bewegungen des Patientenkopfs der Zahnarzt das Beleuchtungsfeld jedes Mal durch entsprechendes Verstellen der Leuchte nachführen muss. Die Größe des zu beleuchtenden Behandlungsfelds ist dabei durch die Vorgabe der Norm ISO968 begrenzt, welche eine maximale Beleuchtungsstärke von 1200 Lux im Abstand von 60mm oberhalb des Zentrums fordert. Hintergrund für diese Vorschrift ist, dass der Patient nicht unnötig geblendet werden soll.

Bei der aus dem Stand der Technik bekannten Leuchte wird das Einhalten der oben erwähnten Blendgrenze in Verbindung mit einem großen, homogen ausgeleuchteten Lichtfeld mit Hilfe der zum Einsatz kommenden optischen Elemente sichergestellt. Im Randbereich des Lichtfelds ergibt sich hierbei ein Übergang von einem Bereich hoher Lichtintensität von etwa 20 kLux zu einem Bereich geringer Lichtintensität von z.B. 2 kLux auf einer sehr kurzen Strecke. Dabei hat sich gezeigt, dass sich ein derartig hoher sog. Helligkeitsgradient ermüdend auf die Augenaktivität des Zahnarztes auswirken kann.

Eine andere Problematik besteht ferner darin, dass bei der projizierten Überlagerung der Beleuchtungsfelder der einzelnen Beleuchtungseinheiten zu dem Gesamtbeleuchtungsfeld in der Objektebene aufgrund von Fertigungsungenauigkeiten, die nicht vollständig zu vermeiden sind, ein Versatz zwischen den einzelnen Beleuchtungsfeldern auftreten kann. Ein derartiger Versatz wird, auch wenn er nur gering ist, durch den Zahnarzt wahrgenommen, und zwar derart, dass er das Gesamtbeleuchtungsfeld nicht mehr als gleichmäßig beleuchtete Fläche empfindet.

Eine weitere gattungsgemäße Leuchte ist aus EP 2 587 128 A1 bekannt. Die hier beschriebene Leuchte weist zumindest ein Leuchtmittel in Form von LEDs, verschiedene optische Mittel zur Strahlbündelung, sowie einen Diffusor auf.

Ausgehend von diesem Stand der Technik liegt der vorliegenden Erfindung deshalb die Aufgabenstellung zugrunde, die Ausgestaltung einer medizinischen Leuchte derart zu verbessern, dass eine bessere und angenehmere Ausleuchtung des Operationsfelds erzielt werden kann.

Die Aufgabe wird durch eine medizinische Leuchte mit den Merkmalen des Anspruchs 1 gelöst. Vorteilhafte Weiterbildungen der Erfindung sind Gegenstand der abhängigen Ansprüche.

Die erfindungsgemäße Lösung sieht vor, zur Vermeidung der oben geschilderten Probleme in den Strahlengang der Beleuchtungseinheit einen Diffusor zu integrieren, der den großen Gradienten vom Beleuchtungsfeld zur nicht beleuchteten Umgebung verringert. Der wahrgenommene Versatz einzelner Beleuchtungsfelder wird mit Hilfe des Diffusors abgeschwächt, was in einer homogeneren, gleichmäßigeren Helligkeitsverteilung im Beleuchtungsfeld resultiert. Gleichzeitig werden starke Kontraste im Randbereich des Beleuchtungsfelds vermieden, was letztendlich zu einer deutlich angenehmeren Ausleuchtung des Operationsfelds führt.

Gemäß der vorliegenden Erfindung wird also eine medizinische Leuchte, insb. eine zahnärztliche Behandlungsleuchte für das intraorale Ausleuchten eines Operationsfelds vorgeschlagen, welche mindestens eine Beleuchtungseinheit mit Leuchtmitteln sowie optischen Mitteln zur Erzeugung eines Lichtfelds in einer Objektebene aufweist, wobei erfindungsgemäß ein Diffusor vorgesehen ist, der dazu ausgebildet ist, das Lichtfeld in der Objektebene aufzuweiten.

Zur Realisierung des erfindungsgemäßen Diffusors bestehen verschiedene Möglichkeiten. Als besonders vorteilhaft hat sich hierbei die Verwendung eines Diffusors mit einer holographischen Struktur (beispielsweise einer sog. µ-Struktur) erwiesen, der zu einem zirkular bzw. elliptisch polarisierten Licht führt und dabei eine große Transmission aufweist. Mit Hilfe eines derartigen Diffusors können nicht nur die oben genannten Probleme durch Aufweiten des Lichtfelds in der Objektebene vermieden werden sondern er bringt ferner auch den Vorteil mit sich, dass eine deutlich geringere Blendung des Arztes vorliegt, da Reflexionen z.B. an den Zahnoberflächen eines Patienten vermieden werden.

Der Diffusor wird auf einer Abdeckscheibe der Leuchte ausgebildet. In der Regel handelt es sich bei diesen Komponenten um Kunststoffelemente, die im Spritzgussverfahren hergestellt werden, d.h., die Diffusorstruktur wird dann in einfacher Weise in das entsprechende Spritzgusswerkzeug integriert, was letztendlich dazu führt, dass der Diffusor sehr kostengünstig hergestellt und in die Leuchte integriert werden kann.

Ein weiterer Vorteil der Integration des Diffusors in das Spritzgusswerkzeug zum Herstellen der Abdeckung besteht darin, dass der Grad der Streuung bzw. der Grad der Zirkulation des Lichts ortsabhängig gestaltet werden kann. Hierdurch kann der Gradient des Übergangs des Beleuchtungsfelds zur Umgebung nochmals besser beeinflusst und systemspezifisch eingestellt werden. Eine andere denkbare Möglichkeit würde auch darin bestehen, den Diffusor mit Hilfe einer geeigneten Erodierstruktur auf einer optisch wirksamen Oberfläche im Strahlengang der Leuchte zu erzeugen.

Wie bereits erwähnt, weist die erfindungsgemäße medizinische Leuchte mehrere Beleuchtungseinheiten auf, welche derart ausgerichtet bzw. ausgebildet sind, dass die entsprechenden Lichtfelder in der Objektebene im Wesentlichen übereinstimmen. Es ist vorgesehen, dass ein gemeinsamer Diffusor für alle Beleuchtungseinheiten eingesetzt wird, der dann in sehr einfacher Weise dadurch realisiert wird, dass die Diffusorstruktur Bestandteil einer gemeinsamen Abdeckung der Leuchte ist.

Letztendlich wird also durch die Erfindung eine medizinische Leuchte zur Verfügung gestellt, welche hinsichtlich ihrer lichttechnischen Eigenschaften nochmals Vorteile gegenüber den aus dem Stand der Technik bekannten Lösungen aufweist.

Nachfolgend soll die Erfindung anhand der beiliegenden Zeichnung näher erläutert werden. Es zeigen:
- Figur 1: die Ansicht einer erfindungsgemäßen zahnärztlichen Behandlungsleuchte;
- Figur 2: die für die Erzeugung des Lichtfelds verantwortlichen Komponenten der Leuchte gemäß Figur 1 in Explosionsdarstellung;
- Figuren 3 und 4: schematische Darstellungen zur Wirkungsweise der optischen Komponenten bei einer Leuchte gemäß dem Stand der Technik;
- Figur 5: die Lichtabgabe bei der erfindungsgemäßen Leuchte und
- Figur 6: einen Vergleich des Intensitätsabfalls im Randbereich des Leuchtfelds bei einer Leuchte gemäß dem Stand der Technik und einer erfindungsgemäßen Leuchte.

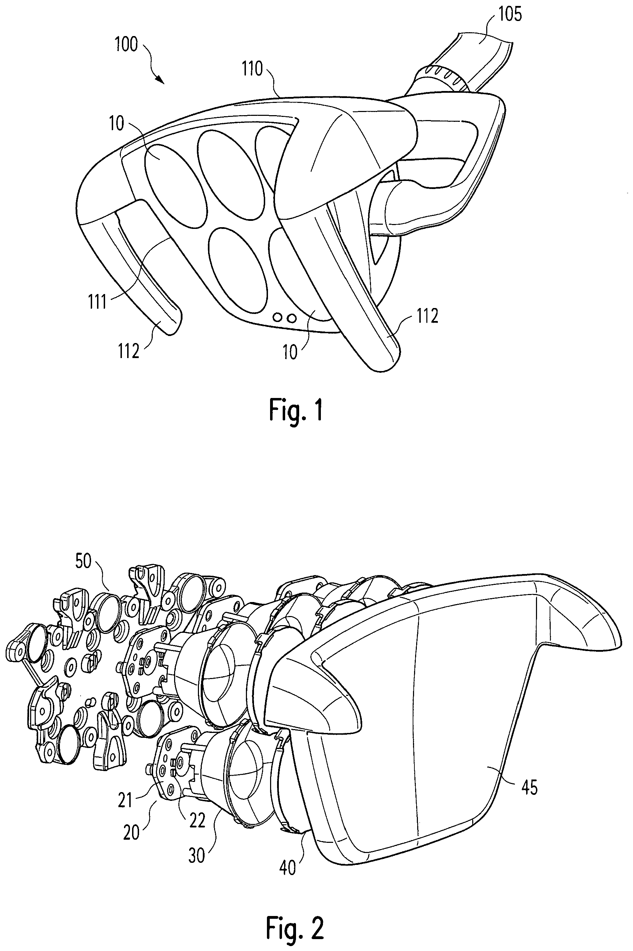

Die in Figur 1 in perspektivischer Ansicht dargestellte, allgemein mit dem Bezugszeichen 100 versehene medizinische Leuchte ist eine zahnärztliche Behandlungsleuchte, mit deren Hilfe das Operationsfeld eines zahnmedizinischen Arbeitsplatzes ausgeleuchtet werden soll. Der Leuchtenkopf 110 ist an einem nicht näher dargestellten Gelenkarm 105 verstellbar angeordnet, derart, dass er in gewünschter Weise auf den Operationsbereich ausgerichtet werden kann. Zur besseren Verstellbarkeit weist hierzu das Gehäuse 111 des Leuchtenkopfs zwei seitliche Griffteile 112 auf, welche eine einfache manuelle Verstellung der Anordnung der Leuchte 100 ermöglichen.

Die Lichterzeugung und Lichtabgabe bei der Leuchte 100 gemäß Figur 1 erfolgt mit Hilfe mehrerer Beleuchtungseinheiten 10, welche jeweils ein Strahlenbündel generieren, das über ein vordere transparente Lichtabstrahlseite der Leuchte 100 abgegeben wird. Im dargestellten Ausführungsbeispiel sind fünf Beleuchtungseinheiten 10 vorgesehen, wobei drei hiervon in einer oberen Reihe und zwei darunterliegend angeordnet sind, derart, dass sich insgesamt gesehen eine leicht trapezförmige Lichtabstrahlfläche ergibt. Allerdings sind auch andere Anordnungen bekannt, bei denen bspw. vier oder fünf Beleuchtungseinheiten nebeneinander liegend angeordnet sind.

Die für die Lichterzeugung und Lichtabgabe wesentlichen Komponenten der Leuchte 100 sind in Explosionsdarstellung in Figur 2 gezeigt. Als Trägerelement 50 dient hierbei eine in dem Gehäuse 111 angeordnete Platine bzw. Leiterplatte, auf der die einzelnen Beleuchtungseinheiten 10 montiert werden. Diese Beleuchtungseinheiten 10 bestehen im dargestellten Ausführungsbeispiel aus drei wesentlichen Komponenten, nämlich den Leuchtmitteln 20, ersten optischen Mitteln zur Strahlbildung 30 sowie zweiten optischen Mitteln zum Erzeugen eines Leuchtfelds 40. Die eigentliche Lichtabgabe erfolgt dann über eine lichtdurchlässige Abdeckscheibe 45, welche den vorderen Lichtabstrahlbereich des Leuchtenkopfs 110 abdeckt und gleichzeitig auch einen Schutz für die im Inneren befindlichen Komponenten darstellt.

Der Aufbau und die Funktionsweise der verschiedenen Komponenten der Beleuchtungseinheiten 10 entspricht im Wesentlichen dem aus der bereits genannten DE 10 2011 075 753 A1 bekannten Aufbau. Dementsprechend soll nachfolgend lediglich kurz die Funktion der verschiedenen Komponenten erläutert werden.

Mit dem Bezugszeichen 20 sind wie bereits erwähnt die Leuchtmittel bezeichnet, die im dargestellten Fall durch LED-Lichtquellen gebildet werden. Genau genommen sind jeweils auf einem Trägerelement in Form einer etwa hexagonalen Trägerplatte 21 ein oder mehrere LEDs 22 angeordnet, welche für die Lichterzeugung verantwortlich sind. Es kann sich wie bereits erwähnt um Einzel-LEDs oder um LED-Arrays handeln, wobei durchaus denkbar wäre, unterschiedliche LEDs miteinander zu kombinieren. Es könnte sich hierbei um LEDs unterschiedlicher Farbe oder Farbtemperatur handeln, wobei deren Licht dann mit den nachfolgend noch näher beschriebenen optischen Mitteln derart vermischt wird, dass letztendlich die Lichtabgabe der Leuchte 100 mit Licht in dem gewünschten Farbton bzw. der gewünschten Farbtemperatur erfolgt. Die Trägerelemente 21 für die LED-Lichtquellen 22 sind hierbei mit dem Träger 50 gekoppelt, wobei insb. spezielle Maßnahmen vorgesehen sein können, durch welche eine effiziente Abführung der während des Betriebs auftretenden Wärme gewährleistet ist.

Im Strahlengang des von den LED-Lichtquellen 22 erzeugten Lichts sind dann zunächst die ersten optischen Mittel 30 angeordnet. Es handelt sich um Mittel zur Kollimation des LED-Lichts, mit deren Hilfe also das von den LED-Leuchtmitteln 22 üblicherweise in einem sehr großen Winkelbereich abgestrahlte Licht zunächst gebündelt wird. Diese optischen Mittel 30 zur Strahlenbündelung können in unterschiedlichster Weise realisiert werden und bspw. wie aus dem Stand der Technik bekannt Reflektorelemente und Linsenelemente beinhalten. Wesentlich ist, dass mit Hilfe dieser ersten optischen Mittel 30 das Licht in ein verhältnismäßig schmales Strahlenbündel zusammengefasst wird, welches dann auf die zweiten optischen Mittel 40 fällt.

Diese zweiten optischen Mittel 40 sind entscheidend dafür verantwortlich, in der Objektebene das angestrebte Lichtfeld zu erzielen. D.h., diese zweiten optischen Mittel 40 lenken die Lichtstrahlen des von dem ersten optischen Mittel 30 erzeugten Strahlenbündels derart um, dass das Licht möglichst gleichmäßig auf einen bestimmten Bereich gerichtet wird, der dann das sog. Leuchtfeld darstellt. Eine denkbare Ausgestaltung dieser zweiten optischen Mittel kann entsprechend der oben beschriebenen DE 10 2011 075 753 A1 z.B. darin bestehen, dass diese durch eine Scheibe gebildet werden, welche eine Vielzahl linsenartiger Elemente aufweist. Die Linsen sind dabei derart ausgestaltet, dass sie selbst das entsprechend auftreffende Teilstrahlenbündel derart umlenken, dass das Lichtfeld erzeugt wird. D.h., durch jede Einzellinse wird sozusagen eine eigenständige virtuelle Lichtquelle gebildet, welche den gewünschten Bereich beleuchtet. Dabei können die Einzellinsen z.B. dann derart gestaltet werden, dass alle hierdurch gebildeten virtuellen Lichtquellen einen identischen Bereich beleuchten, so dass also durch Überlagerung einer Vielzahl einzelner Strahlenbündel eine möglichst gleichmäßige Ausleuchtung erzielt wird. Ferner kann bei dieser Art der Beleuchtung eines gemeinsamen Bereichs durch eine Vielzahl einzelner virtueller Lichtquellen eine Schattenbildung reduziert werden. Wie bereits erwähnt, könnten allerdings die zweiten optischen Mittel 40 auch anderweitig gestaltet sein, so dass z.B. die virtuellen Lichtquellen versetzt überlappende Strahlenbündel generieren, welche dann allerding wieder gemeinsam das Lichtfeld ausleuchten. Ferner wäre es auch denkbar, die ersten und zweiten optischen Mittel 30 und 40 in einer gemeinsamen Baueinheit zusammenzufassen.

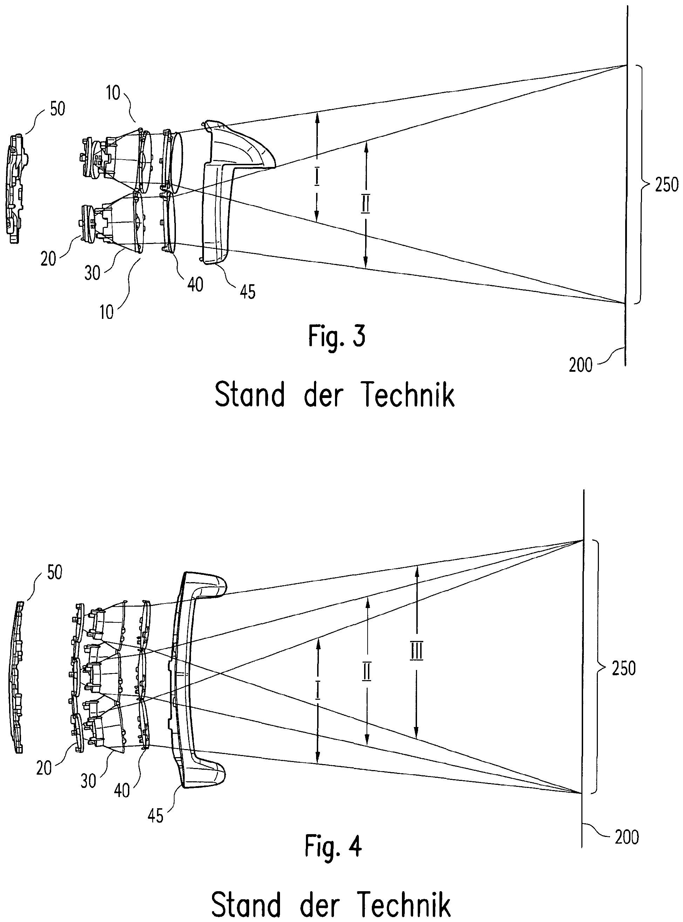

Das Überlagern einzelner Strahlenbündel erfolgt im Übrigen nicht nur im Hinblick auf die Wirkungsweise der zweiten optischen Mittel 40, sondern insbesondere auch bzgl. der verschiedenen Beleuchtungseinheiten 10. Dargestellt ist dies zunächst in den Figuren 3 und 4 anhand der aus dem Stand der Technik bekannten Leuchte, wobei in Figur 13 ist in seitlicher Darstellung die Überlagerung der Strahlenbündel zweier übereinander angeordneter Beleuchtungseinheiten 10 gezeigt ist. Erkennbar ist dabei, dass zunächst durch die ersten optischen Mittel 30 das Licht jeweils in ein nahezu paralleles Strahlenbündel umgeformt wird. Die zweiten optischen Mittel 40 jedoch weiten dann das jeweilige Strahlenbündel auf, wobei die Anordnung und Ausrichtung der verschiedenen optischen Komponenten der beiden Beleuchtungseinheiten 10 derart ist, dass sich beide letztendlich abgegebenen Strahlenbündel I und II in einer Objektebene 200 im Wesentlichen identisch überlagern. Hierdurch wird das schematisch angedeutete Lichtfeld 250, welches die Gesamtlichtabgabe der Leuchte 100 charakterisiert, definiert.

Dieses Überlagern der verschiedenen Strahlenbündel der Beleuchtungseinheiten 10 erfolgt dabei auch in einer Ebene senkrecht zu der Darstellung gemäß Figur 3, wie in Figur 4 gezeigt ist. Hier ist das Überlagern der drei in der oberen Reihe angeordneten Beleuchtungseinheiten 10 dargestellt, wobei wiederum in der Objektebene 200, die bspw. in einem Abstand von 70cm vor der Leuchte 100 angeordnet ist, alle drei Strahlenbündel I, II und III im Wesentlichen identisch überlagern.

Die bisherigen Erläuterungen treffen insbesondere auch auf die bereits aus dem Stand der Technik bekannte Leuchte zu. Es hat sich allerdings gezeigt, dass hier die letztendlich erzielbare Beleuchtung noch weiter optimiert werden kann.

Ein erstes Problem besteht bspw. darin, dass im Randbereich des Lichtfelds 250 ein verhältnismäßig starker Helligkeitsabfall stattfindet. Dies führt zu sehr starken Kontrasten bei der Beleuchtung, welche sich ermüdend insb. auf den Zahnarzt und/oder die Zahnarzthelferin auswirken können. Ein weiteres Problem besteht darin, dass eine 100%tig exakte Überlagerung der einzelnen Lichtbündel der Beleuchtungseinheiten 10 aufgrund von Fertigungstoleranzen und dergleichen nicht erzielt werden kann. Ein gewisser Versatz bei der Generierung des Lichtfelds 250 in der Objektebene 200 wird zwangsläufig vorliegen, der dann letztendlich als eine nichtgleichmäßige Ausleuchtung des Operationsfelds wahrgenommen wird.

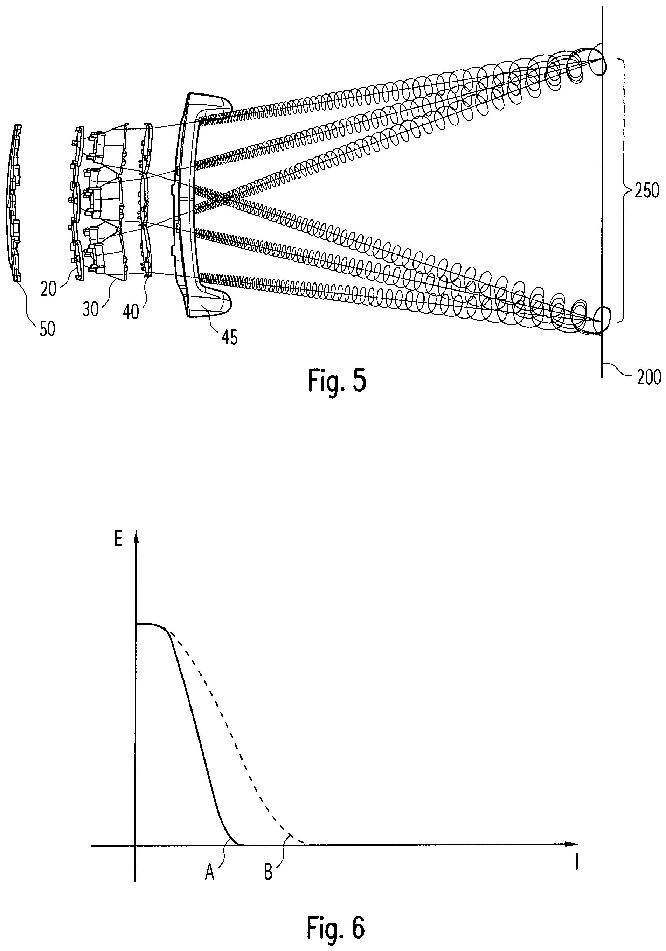

Um diese Probleme zu vermeiden, wird erfindungsgemäß vorgeschlagen, in den Strahlengang der Lichtabgabe einen Diffusor zu integrieren. Bei dem Ausführungsbeispiel einer erfindungsgemäßen Leuchte, wie es in Figur 5 dargestellt ist, ist hierzu vorgesehen, den Diffusor in die lichtdurchlässige Abdeckung 45 der Leuchte zu integrieren. Die Darstellung von Figur 5 entspricht hierbei der Darstellung von Figur 4, wobei nunmehr allerdings schematisch angedeutet ist, dass aufgrund der lichtstreuenden Eigenschaften der Abdeckung 45 die einzelnen Strahlenbündel leicht aufgeweitet werden. Im Vergleich zu der Darstellung gemäß den Figuren 3 und 4, bei denen davon ausgegangen wurde, dass die Abdeckung 45 durch eine plane, insb. durch eine nicht-lichtstreuende Scheibe gebildet wird, werden hierdurch also die Randbereiche der sich überlagernden Lichtbündel leicht vergrößert. Es handelt sich hierbei um eine lediglich schwache Streuung, die zusätzlich in die Beeinflussung der Lichtabgabe mit aufgenommen wird. Diese leichte Streuung hat allerdings nunmehr zur Folge, dass sich ein leicht vergrößertes Lichtfeld 250 ergibt, welches allerdings aufgrund der Herabsetzung der Kontraste bzw. des Helligkeitsgradienten im Randbereich als gleichmäßiger und angenehmer beleuchtet wahrgenommen wird. Ferner wirken sich die reduzierten Kontraste im Randbereich auch weniger ermüdend auf die Augentätigkeit bspw. des Zahnarztes aus.

Diese Reduzierung des Helligkeitsgradienten ist schematisch in Figur 6 dargestellt, welche den Abfall der Helligkeit im Randbereich des Lichtfelds bei der Leuchte gemäß dem Stand der Technik (Kurve A) sowie im Vergleich dazu den Abfall bei der erfindungsgemäßen Leuchte (Kurve B) zeigt. Deutlich ist erkennbar, dass der Randbereich bei der erfindungsgemäßen Leuchte also leicht "verschwommen" ist. Die führt dazu, dass leichte Abweichungen in der Ausrichtung der verschiedenen Leuchteneinheiten, die wie bereits erwähnt ohnehin kaum zu vermeiden sind, nun nicht mehr als Schattierungen im Randbereich des Lichtfelds wahrgenommen werden. Allerdings wird durch diese Maßnahme nicht nur eine Verbesserungen der Beleuchtungssituation im Randbereich des Lichtfeldes erzielt sonder zusätzlich auch der Effekt erreicht, dass sich über das Gesamte Lichtfeld hinweg ein deutlich gleichmäßigeres Erscheinungsbild ergibt.

Die Realisierung des Diffusors besteht wie bereits erwähnt darin, diesen in die Abdeckung 45 zu integrieren. Da es sich bei der Abdeckung 45 in der Regel um ein Kunststoffteil handelt, welches im Spritzgussverfahren hergestellt wird, besteht hierbei die Möglichkeit, das entsprechende Werkzeug zur Herstellung der Abdeckung bereits entsprechend zu gestalten, sodass z.B. an der den Lichtquellen zugewandten Innenseite der Abdeckung 45 geeignete lichtstreuende Strukturen ausgebildet sind. Dies stellt also eine sehr kostengünstige und dennoch effiziente Vorgehensweise dar, um die Leuchten in erfindungsgemäßer Weise zu gestalten.

Es hat sich dabei als vorteilhaft herausgestellt, wenn der Diffusor mit Hilfe einer holographischen Struktur, insb. einer sog. µ-Struktur realisiert wird. Diese Struktur kann dabei derart ausgebildet sein, dass das hindurchtretende Licht zirkular bzw. elliptisch polarisiert wird, wobei der Diffusor trotz allem einen hohen Transmissionsgrad aufweist. Das Polarisieren des hindurchtretenden Lichts führt dabei zu dem zusätzlichen vorteilhaften Effekt, dass Reflexionen bspw. an der Zahnoberfläche reduziert werden und dementsprechend eine deutlich geringere Gefahr der Blendung des Arztes besteht.

Alternativ zu der vorgeschlagenen Vorgehensweise könnte der Diffusor nicht erfindungsgemäß allerdings bspw. auch Bestandteil der zweiten optischen Mittel 40 sein. Auch in diesem Fall würde die Möglichkeit bestehen, das wiederum vorzugsweise im Spritzgussverfahren hergestellte entsprechende optische Element bereits mit den lichtstreuenden Eigenschaften zu versehen.

Schließlich würde auch noch die nicht erfindungsgemäße Möglichkeit bestehen, den Diffusor als separates Bauelement in die Leuchte 100 einzubringen. In diesem Fall könnte dann eine entsprechende flexible oder starre Folie zum Einsatz kommen, welche vorzugsweise dann zwischen den zweiten optischen Mitteln 40 und der Abdeckung 45 angeordnet wird.

Letztendlich kann also die erfindungsgemäße Lösung mit Hilfe relativ einfach durchzuführender Maßnahmen realisiert werden. Trotz allem wird mit Hilfe dieser Maßnahmen die Lichtabgabe der Leuchte signifikant verbessert. Dabei wirken sich die Vorteile des Diffusors bereits auf die Lichtabgabe einer einzelnen Beleuchtungseinheit aus.

## Patentansprüche

1. Medizinische Leuchte (100), insbesondere zahnärztliche Behandlungsleuchte für das intraorale Ausleuchten eines Operationsfelds, aufweisend mindestens eine Beleuchtungseinheit (10) mit
Leuchtmitteln (20) und optischen Mitteln (40) zur Erzeugung eines Lichtfelds (250) in einer Objektebene (200) sowie
einen Diffusor, der dazu ausgebildet ist, das Lichtfeld (250) in der Objektebene (250) aufzuweiten,
wobei die Leuchte (100) mehrere Beleuchtungseinheiten (10) aufweist, welche derart ausgerichtet bzw. ausgebildet sind, dass die entsprechenden Lichtfelder im Wesentlichen übereinstimmen,
**dadurch gekennzeichnet, dass** der Diffusor auf einer Abdeckscheibe (45) der Leuchte (100) ausgebildet ist.

2. Medizinische Leuchte nach Anspruch 1,
**dadurch gekennzeichnet,**
**dass** die Abdeckscheibe (45) als ein Kunststoffelement ausgebildet ist, welches im Spritzgussverfahren mit einem Spritzgusswerkzeug hergestellt wird, so dass bei der Herstellung der Abdeckung (45) an der den Leuchtmitteln (20) zugewandten Innenseite der Abdeckung (45) geeignete lichtstreuende Strukturen ausgebildet werden.

3. Medizinische Leuchte nach Anspruch 1 oder 2,
**dadurch gekennzeichnet,**
**dass** der Diffusor durch eine µ-Struktur gebildet ist.

4. Medizinische Leuchte nach Anspruch 3,
**dadurch gekennzeichnet,**
**dass** die µ-Struktur ortsabhängig unterschiedlich ausgeführt ist.

5. Medizinische Leuchte nach Anspruch 3,
**dadurch gekennzeichnet,**
**dass** die µ-Struktur eine holographische Struktur ist.

6. Medizinische Leuchte nach einem der vorherigen Ansprüche,
**dadurch gekennzeichnet,**
**dass** die optischen Mittel der Beleuchtungseinheit (10) erste optische Mittel (30) zur Strahlbündelung sowie zweite optische Mittel (40) zur Erzeugung des Lichtfelds (250) umfassen.

7. Medizinische Leuchte nach Anspruch 6,
**dadurch gekennzeichnet,**
**dass** die zweiten optischen Mittel (40) eine Vielzahl von Linsen umfassen, welche vorzugsweise auf einer gemeinsamen Scheibe angeordnet sind.

8. Medizinische Leuchte nach Anspruch 7,
**dadurch gekennzeichnet,**
**dass** jede der Linsen dazu ausgebildet ist, das durch sie hindurchtretende Teilstrahlenbündel in der Objektebene auf das Lichtfeld zu (250) zu projizieren.

9. Medizinische Leuchte nach Anspruch 7,
**dadurch gekennzeichnet,**
**dass** die Linsen dazu ausgebildet ist, das durch sie hindurchtretende Teilstrahlenbündel derart umzulenken, dass die auf die Objektebene projizierten Strahlenbündel in der Gesamtheit das Lichtfeld (250) ergeben.

10. Medizinische Leuchte nach einem der vorherigen Ansprüche,
**dadurch gekennzeichnet,**
**dass** die Leuchtmittel (20) eine LED (22) oder ein LED-Array aufweisen.

## Claims

1. Medical lamp (100), in particular dental treatment lamp for the intraoral illumination of an operation field, comprising at least one illumination unit (10) having
lighting means (20) and optical means (40) for producing a light field (250) in an object plane (200) and a diffusor embodied to expand the light field (250) in the object plane (250),
wherein the lamp (100) has a plurality of illumination units (10) that are aligned or embodied in such a way that the corresponding light fields substantially correspond,
**characterized in that**
the diffusor is embodied on a covering panel (45) of the lamp (100).

2. Medical lamp according to Claim 1,
**characterized**
**in that** the covering panel (45) is embodied as a plastic element which is produced in an injection moulding method using an injection mould such that, when the cover (45) is produced, suitable light-scattering structures are formed on the inner side of the cover (45) that faces the lighting means (20) .

3. Medical lamp according to Claim 1 or 2,
**characterized**
**in that** the diffusor is formed by a microstructure.

4. Medical lamp according to Claim 3,
**characterized**
**in that** the microstructure has a different embodiment depending on location.

5. Medical lamp according to Claim 3,
**characterized**
**in that** the microstructure is a holographic structure.

6. Medical lamp according to any one of the preceding claims,
**characterized**
**in that** the optical means of the illumination unit (10) comprise first optical means (30) for beam focusing and second optical means (40) for producing the light field (250).

7. Medical lamp according to Claim 6,
**characterized**
**in that** the second optical means (40) comprise a multiplicity of lenses which are preferably arranged on a common panel.

8. Medical lamp according to Claim 7,
**characterized**
**in that** each of the lenses is embodied to project the partial beam passing therethrough onto the light field (250) in the object plane.

9. Medical lamp according to Claim 7,
**characterized**
**in that** the lenses are embodied to deflect the partial beam passing therethrough in such a way that the totality of the beams projected onto the object plane yield the light field (250).

10. Medical lamp according to any one of the preceding claims,
**characterized**
**in that** the lighting means (20) comprise an LED (22) or an LED array.

## Revendications

1. Lampe (100) à usage médical, en particulier lampe de traitement à usage dentaire destinée à l'éclairage intra-oral d'un champ opératoire, ladite lampe comprenant au moins une unité d'éclairage (10), munie de moyens d'éclairage (20) et de moyens optiques (40) destinés à générer un champ de lumière (250) dans un plan d'objet (200), et un diffuseur conçu pour élargir le champ de lumière (250) dans le plan d'objet (250),
la lampe (100) comprenant une pluralité d'unités d'éclairage (10) orientées ou conçues de telle sorte que les champs de lumière correspondants coïncident sensiblement,
**caractérisée en ce que** le diffuseur est formé sur un disque de recouvrement (45) de la lampe (100).

2. Lampe à usage médical selon la revendication 1,
**caractérisée en ce que**
le disque de recouvrement (45) est réalisé sous la forme d'un élément en matière plastique fabriqué par un procédé de moulage par injection au moyen d'un outil de moulage par injection de manière à former, lors de la fabrication du recouvrement (45), des structures de diffusion de lumière appropriées du côté intérieur du recouvrement (45) qui fait face aux moyens d'éclairage (20).

3. Lampe à usage médical selon la revendication 1 ou 2,
**caractérisée en ce que**
le diffuseur est formé par une µ-structure.

4. Lampe à usage médical selon la revendication 3,
**caractérisée en ce que**
la µ-structure est réalisée de manière différente selon l'endroit.

5. Lampe à usage médical selon la revendication 3,
**caractérisée en ce que**
la µ-structure est une structure holographique.

6. Lampe à usage médical selon l'une des revendications précédentes,
**caractérisée en ce que**
les moyens optiques de l'unité d'éclairage (10) comprennent des premiers moyens optiques (30) destinés à la focalisation de faisceaux et des deuxièmes moyens optiques (40) destinés à générer le champ de lumière (250) .

7. Lampe à usage médical selon la revendication 6,
**caractérisée en ce que**
les deuxièmes moyens optiques (40) comprennent une pluralité de lentilles qui sont disposées de préférence sur un disque commun.

8. Lampe à usage médical selon la revendication 7,
**caractérisée en ce que**
chacune des lentilles est conçue pour projeter des sous-faisceaux, qui la traversent, en direction du champ de lumière (250) dans le plan d'objet.

9. Lampe à usage médical selon la revendication 7,
**caractérisée en ce que**
les lentilles sont conçues pour rediriger des sous-faisceaux qui les traversent de telle sorte que les faisceaux de rayons, projetés sur le plan de l'objet, produisent dans l'ensemble le champ de lumière (250).

10. Lampe à usage médical selon l'une des revendications précédentes,
**caractérisée en ce que**
les moyens d'éclairage (20) comprennent une LED (22) ou un agencement de LED.
